# EUROPEAN PATENT SPECIFICATION

(11) **EP 0 937 468 B1**
(45) Date of publication and mention of the grant of the patent: **30.09.2009**
(21) Application number: 98116437.9
(22) Date of filing: 31.08.1998
(51) Int. Cl.: A61L 24/04

(54) **Adhesive composition for surgical use application**
Klebstoffzusammensetzung für chirurgische Zwecke
Composition adhésive à usage chirurgical

(30) Priority: 12.02.1998 JP 6920398
(43) Date of publication of application: 25.08.1999
(73) Proprietor: BMG INCORPORATED, Kyoto-shi, Kyoto-fu 601-8023 (JP)
(72) Inventor: Gen, Shokyu, Uji-shi, Kyoto 611 (JP); Nakajima, Naoki, Muko-shi, Kyoto 617-0002 (JP)
(74) Representative: Albrecht, Thomas

(56) References cited:
- US-A- 3 527 841
- US-A- 5 328 687
- DATABASE WPI Section Ch, Week 199603 Derwent Publications Ltd., London, GB; Class A14, AN 1988-364119 XP002146295 & JP 07 116409 A (BIOMATERIALS UNIVERS) 13 December 1995 (1995-12-13)

## Description

The present invention relates to adhesive compositions for surgical use application, more particularly novel compositions for adjusting a suitable viscosity, reducing a cytotoxicity, having a softness similar to a soft tissue of a living body organism, easily biodegrading and bioabsorbing into the living body organism at a moderate velocity after healing by improving α-cyanoacrylate adhesive composition.

Suturing and anastomosis of the wound is one of most important operating techniques in any surgical operation. Therefore, the healing velocity of the wound largely makes the difference between a good or a poor operation. Nowadays, the material compositions and operating methods for improving healing velocity are continuously improving, including methods for suturing the wound, and many good sutures have become readily available as a result. However, special techniques are still necessary in operations involving microvascular anastomosis and nerve sutures. Therefore, new invention systems concerning adhesive methods in the living body system are needed in order to proceed the surgical operations in a short time. There are many proposed reports and inventions all over the world for the above purpose.

The α-cyanoacrylate adhesive compositions are already and widely sold in the market as adhesive compositions for surgical use application, but they still remain in limited use in special clinical applications because they often flood to unnecessary or unexpected parts because of the low viscosity of the composition, sometimes damaging the soft tissue and especially showing an excellent cytotoxicity for the living cell in addition.

The inventors have already disclosed several means in an invention (JP. Pat. 116409/1995) in order to remedy the above defects. This application concerns new compositions for surgical use application, in which a homo-polymer of lactic acid (DL-, D- and L-type), a co-polymer of lactic acid and glycolic acid or a co-polymer of lactic acid and ε-caprolactone, which is easily biodegraded and bioabsorbed into the living body organism, is added to the α-cyanoacrylate adhesive composition of the surgical use as a thickening agent and a stabilizer.

However, this application fails to disclose the cytotoxicity of the surgical adhesive composition for the living cell, and therefore, the cytotoxicity for the living cell is still uncertain. Weight-average molecular weights of the homo-polymer of DL-lactic acid and the co-polymer of the lactic acid and ε-caprolactone are about 140,000 and 220,000 respectively, which is in a relative high molecular weight category and so can be employed as the thickening agent. They also fail to disclose the molecular weight effects on plasticizing after hardening, and the hydrolyzing velocity of co-polymer compositions of lactic acid and ε-caprolactone when employed as a thickening agent.

U.S. patent 5,328,687 describes a biocompatible monomer composition containing, for example, an α-cyanoacrylate and a formaldehyde scavenger compound for use as an in vivo tissue adhesive. The composition may further contain, inter alia, thickening agents including, for example, lactic acid-caprolactone copolymers.

U.S. patent 3,527,841 describes α-cyanoacrylate adhesive compositions for general and particularly for surgical use containing poly-(lactic acid) as a viscosity modifier and an acidic component such as SO₂ and/or a free radical scavenger such as hydroquinone as a polymerization inhibitor.

The present invention proposes a novel adhesive composition for surgical use application adding a polymer characteristic to be biodegraded and bioabsorbed into the living body organism to an α-cyanoacrylate adhesive composition. The polymer is a co-polymer of DL-lactic acid and an ε-caprolactone.

The ratio of composition and the weight-average molecular weight of the co-polymer of DL-lactic acid and ε-caprolactone are in the ranges of 70:30 to 30:70 and of 10,000 to 120,000, respectively, and it is preferred that the concentration of the co-polymer is in the range of 1 to 50 weight by weight percent.

The novel adhesive compositions for surgical use application of the invention can be easily adjusted to any degree of thickening agent as a result of the possibility to control viscosity. The hardened polymer after a polymerization shows a softness and has an affinity with the living body organism such as blood vessels, skin and viscera. It is possible that wider applications can be added to former uses of the application of α-cyanoacrylate because the composition is widely applied to a hemostasis, and possibly certificated a part and a volume of the wound by staining dyes.

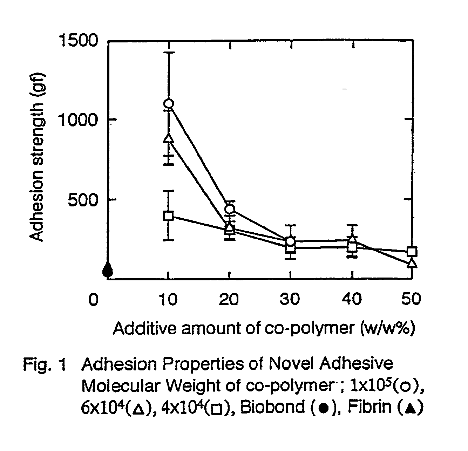

Fig.1 is a schematic view illustrating the adhesion properties between swine skin and n-butyl-cyanoacrylate (NBCA) specimen by adding polymers of various weight-average molecular weights of DL-lactic acid and ε-caprolactone (mole ratio of 50:50 by DL-lactic acid and ε-caprolactone), and also illustrating adhesion strengths of "Biobond" (fabricated by Yoshitomi Ph.) and "Fibrin Glue" (fabricated by Fujisawa Ph.) in comparison with adhesives sold in the market.

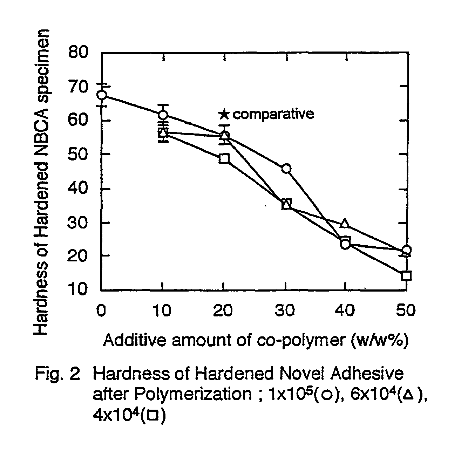

Fig.2 is a schematic view illustrating the hardness of a hardened NBCA specimen after the polymerization according to the addition of various weight-average molecular weights of DL-lactic acid and ε-caprolactone (mole ratio of 50:50 by DL-lactic acid and ε-caprolactone). A vertical axis shows Shore Hardness of D-type.

The present invention relates to adhesive compositions for surgical use application, more particularly new compositions for adjusting a suitable viscosity, reducing cytotoxicity of a cell, having a softness similar to the soft tissue of a living body organism, being easily biodegradable and bioabsorbable into the living body organism at a moderate velocity after healing by improving α-cyanoacrylate adhesive composition.

Essentially, healing the wound of the living body organism completes via a hyperplasia of the connective tissue by a renaturation ability in the living body organism. Therefore, it is preferable to apply an adhesive rather than a suture to fix and support the connective tissue in order not to prevent the restoration ability of the living body organism during the week or 10 days necessary to finally heal the wound. In other words, the role of the preferred adhesive is to fix and adhere the wound in the period needed to complete the healing, and which is then easily biodegraded, and bioabsorbed into the living body organism after healing and which disappears soon thereafter. In addition, it is also important and necessary that biodegraded products of the adhesive are not poisonous or dangerous. We point out the preferable qualities as required of an adhesive in the living body organism as follows.
1) Possibility to adhere at room temperature (bodily temperature) under conditions of a high humidity and contact with fat.
2) When adhering to the face of a wound, combining enough tensile strength with softness in the hardened product.
3) Showing no tissue toxicity and no carcinogenesis, and causing little reactionary resistance as a foreign body.
4) Not interfering with the healing process of a wound in the living organism.
5) The adhesive will biodegrade and be expelled through the living organism after healing.
6) Can be sterilized.
7) The adhesive solution can be easily manipulated and quickly applied to the wound.

The present invention relates to novel compositions for surgical use application satisfying the above conditions for adjusting a suitable viscosity, reducing cytotoxicity for the living cell, having a softness similar to the soft tissue of the living body organism, being easily biodegradable and bioabsorbable into the living body organism at moderate velocity after healing by improving α-cyanoacrylate adhesive composition.

In order to meet the above criteria, the present application is based on the fact that the novel adhesive compositions for surgical use application are fabricated by adding polymers which are easily biodegradable and bioabsorbable into the living body organism to an α-cyanoacrylate adhesive composition, said polymers being the co-polymer of DL-lactic acid and ε-caprolactone.

A most important factor for the surgical use application of the adhesive concerns the cytotoxicity of the living cell, namely safety for the living cell. Therefore, every adhesive has to be reduced to the level of cytotoxicity of the living cell when applied as a surgical adhesive. Moreover, it is difficult to increase the additive amount and adjust to a suitable degree the thickening agent by adding polymers, characterized by being easily biodegraded and bioabsorbed into the living body organism, when the polymers have a weight-average molecular weight of more than 140,000, because the additive amount depends on the molecular weight. Therefore, according to the present invention, a copolymer of DL-lactic acid and ε-caprolactone has a weight-average molecular weight within a range from 10,000 to 120,000. A co-polymer having the weight-average molecular weight of less than 10,000 shows poor properties as a polymer such as an adhesive while the additive amount is possible to increase. Furthermore, it is difficult to get a soft hardened polymer with a high velocity of biodecomposition and bioabsorption into the living body organism when employing high weight-average molecular weight polymer. As mentioned above, velocity largely depends upon the molecular weight and crystallinity. It is preferable to employ a polymer having low molecular weight and crystallinity in order to improve the properties of biodecomposition and bioabsorption. In the case of a copolymer of DL-lactic acid and ε-caprolactone, the glass transition temperature largely depends on the composed ratio of the co-polymer. Therefore, when the DL-lactic acid ratio in the co-polymer is more than 70%, the glass transition temperature of the co-polymer shows therefore a higher value than 0°C. On the other hand, when the ε-caprolactone ratio in the co-polymer is more than 70%, the glass transition temperature of the co-polymer shows a temperature lower than 0°C, but the co-polymer changes hardness according to the crystallinity degree. In conclusion, the ratio of the co-polymer composition is a range from 70:30 to 30:70 from the view of the crystallinity and the softness of the co-polymer.

It is preferred that the composed ratio is within the range for preventing the crystallization of the co-polymer.

In this invention, the co-polymers employed to improve the viscosity and the softness after hardening α-cyanoacrylate are synthesized from lactic acid as a raw material which widely disperses not only in the living body but also in nature, and ε-caprolactone which is widely used as a raw material for polyurethane, available in both household and industry. Those raw materials are ordinarily eliminated in the metabolism of the living body and show no poisonous qualities. Furthermore, co-polymers of DL-lactic acid and ε-caprolactone are already employed as sutures in medical use applications.

The co-polymer of DL-lactic acid and ε-caprolactone employed in the invention is soft even at room temperature and looks like rubber because the weight-average molecular weight is in a relatively low range of 10,000 to 120,000. Not only is the hardened co-polymer soft, but also the adhesive viscosity can be changed to any level by adding α-cyanoacrylate. It is possible to reduce the usage of poisonous α-cyanoacrylate when adjusting to an intensive softness and viscosity by employing as thickening agent a relative low molecular co-polymer having a weight-average molecular weight of less than 120,000 and thereby reducing the cytotoxicity of the living cell to the level of the copolymer of polyethylene glycol.

### EXAMPLES

### Example 1

Fig.1 is a schematic view illustrating the adhesion properties between swine skin (about 1x3 cm) and an NBCA specimen by adding polymers of various weight-average molecular weights of DL-lactic acid and ε-caprolactone (a molecular ratio of 50:50 of DL-lactic acid and ε-caprolactone at a transition temperature of about -8°C). The adhesion property was measured as follows.
1) First, the adhesives of about 10 µl were applied to the swine skin surface (about lxl cm),
2) The treated swine skin surface was adhered to an untreated swine skin surface,
3) A load of about 500 g was applied to the swine skin surfaces and left for 1 minute,
4) The peering strength of the two surfaces was measured at a rate of 10 mm/min. by a tensile tester (fabricated by Shimazu Co.) to estimate adhesion strength.

Fig.1 also illustrates adhesion strengths of "Biobond" (fabricated by Yoshitomi Ph.) and "Fibrin Glue" (fabricated by Fujisawa Ph.) in comparison with adhesives sold in the market. The adhesion strengths gradually decrease in accordance with an increase of additive amounts of co-polymer and a decrease in molecular weight, but even so results are excellent in comparison with those of the adhesives sold in the market.

### Example 2

The following Table 1 shows the initial viscosity (CP, centipoise) of an NBCA specimen after addition of a co-polymer of about 100,000 weight-average molecular weight of DL-lactic acid and ε-caprolactone (a mole ratio of 50:50 by DL-lactic acid and ε-caprolactone).

**Table 1. Viscosity of Novel adhesive**

| | | Viscosity (CP) |
|---|---|---|
| Aron-Alpha | | 59 |
| Biobond | | 3,560 |
| NBCA + co-polymer | | |
| co-polymer | 10% | 843 |
| | 15% | 7,560 |
| | 20% | 30,000 |

The viscosity was measured at 25°C by a B-type rotating viscometer (fabricated by Tokyo Keiki). The viscosity of the adhesives shows excellent value in comparison with those of "Biobond" or "Aron-alpha" (fabricated by Sankyo Ph.).

### Example 3

Fig.2 is a schematic view illustrating the hardness of a hardened NBCA specimen after polymerization after addition of various weight-average molecular weights of DL-lactic acid and ε-caprolactone (a mole ratio of 50:50 DL-lactic acid and ε-caprolactone). The hardness was measured as follows.
1) The specimens were fabricated by polymerization and hardening of the co-polymer at 3 days x 60°C in a vessel containing water.
2) They were pressed at 140°C by hot pressing.
3) A disc of 30 mm diameter and 5 mm thickness was prepared.
4) The hardness of the specimen was measured by a Shore Hardness Tester of a D-type (fabricated by Kohbunshi Keiki).

The hardness shows 68 in the case of no addition of copolymer, but gradually lowers according to the increase in the amount of co-polymer added, and according to the decrease of the co-polymer molecular weight of same added amount. The adhesives remain suitably flexible even after hardening.

### Example 4

The following Table 2 shows test data concerning the cytotoxicity for the living cell of an NBCA specimen after adding co-polymers of DL-lactic acid of about 60,000 weight-average molecular weights and ε-caprolactone (a mole ratio of 50:50 DL-lactic acid and ε-caprolactone at a glass transition temperature of about -9°C). NR50 was defined as reagent concentration value when dropping to a half of the original cytoactivity value, and therefore high toxicity means low NR50 value.

**Table 2. Cytotoxicity of Novel Adhesive**

| | | NR50(ppm) |
|---|---|---|
| Histoacryl | | |
| | Composite | 611± 6 |
| | Hardened Composite | 994± 6 |
| NBCA + co-polymer | | |
| co-polymer 10% | | |
| | Composite | 744± 6 |
| | Hardened Composite | 1,098±29 |
| co-polymer 30% | | |
| | Composite | 967± 9 |
| | Hardened Composite | 1,553±12 |

The test method is described as follows.
1) The hardened co-polymers and their blends of 0.5 g were added to a phosphoric acid buffer solution of 3 ml.
2) They were heated 1 hour at 120°C.
3) The extracted solution from the specimen was subjected to a poison test.
4) The said test procedure followed the literature (J. Biomed. Mater. Res., 29, 829-834 (1995)) and the cytotoxicity was evaluated as amounts of Neutral Red diffused into the cell.

All cytotoxicity data on the specimens, namely their component elements, their co-polymers and their blends are shown in Table 2 and compared with the data of an n-butyl-cyanoacrylate of "Histoacryl" (fabricated by B.Braun and sold in the market). It is clear that all values of the specimen are far lower than "Histoacryl".

The hardened adhesives prepared by the method described in Example 1 were implanted on the back subcutaneous tissue of 20-week-old rabbits. When the implanted part was observed after 3 months, the adhesives were perfectly biodegraded and bioabsorbed and there was no tissue reaction in the ambient parts owing to the adhesive composition.

### Comparative Example

Co-polymers of DL-lactic acid and ε-caprolactone (a mole ratio of 80:20 DL-lactic acid and ε-caprolactone of about 220,000 weight-average molecular weights at a transition temperature of about -8°C) were added to an NBCA as a thickening agent to fabricate the hardened adhesives as described in Example 3. The Shore Hardness was measured and it showed a high value of about 63 as described in Fig. 2. The NR50 of the adhesive showed a high cytotoxicity value of 998+-13 (ppm) as shown in Table 2 following the test method described in Example 4.

Said adhesives were implanted on the back subcutaneous tissue of 20-week-old rabbits. When the implanted part of the tissue was observed after 3 months, about 20 weight by weight percent of said adhesives still remained in the implanted part, which means it was too slowly biodegraded and bioabsorbed. Inflammation was observed in ambient tissue owing to the adhesive composition.

## Claims

1. An adhesive composition for use in surgical applications comprising an α-cyanoacrylate adhesive composition and a biodegradable and bioabsorbable co-polymer of DL-lactic acid and an ε-caprolactone, wherein the molar ratio of DL-lactic acid and ε-caprolactone in the co-polymer is in the range from 70:30 to 30:70 and wherein the weight average molecular weight of the co-polymer is from 10,000 to 120,000.

2. The composition according to claim 1 wherein the concentration of the co-polymer is in the range from 1 to 50 weight percent.

## Patentansprüche

1. Klebstoffzusammensetzung zur Verwendung in chirurgischen Anwendungen, umfassend eine α-Cyanoacrylat-Klebstoffzusammensetzung und ein biologisch abbaubares und bioabsorbierbares Copolymer von DL-Milchsäure und einem ε-Caprolacton, wobei das Molverhältnis von DL-Milchsäure und ε-Caprolacton in dem Copolymer im Bereich von 70 : 30 bis 30 : 70 liegt und wobei das gewichtsmittlere Molekulargewicht des Copolymers von 10.000 bis 120.000 ist.

2. Zusammensetzung gemäß Anspruch 1, wobei die Konzentration des Copolymers im Bereich von 1 bis 50 Gew.-% liegt.

## Revendications

1. Composition adhésive à usage chirurgical comprenant une composition adhésive à base d'alpha-cyanoacrylate et un copolymère biodégradable et bioabsorbable constitué de d,l-acide lactique et d' ε-caprolactone, dans laquelle le rapport molaire du d,l-acide lactique et de l' ε-caprolactone dans le copolymère est compris dans la gamme de 70:30 à 30:70 et dans laquelle le poids moléculaire moyen pondéral est de 10 000 à 120 000.

2. Composition selon la revendication 1, dans laquelle la concentration du copolymère est comprise dans la gamme de 1 à 50% en poids.
